# EUROPEAN PATENT SPECIFICATION

(11) **EP 2 015 791 B1**
(45) Date of publication and mention of the grant of the patent: **20.03.2013**
(21) Application number: 07728903.1
(22) Date of filing: 08.05.2007
(51) Int. Cl.: A61L 27/30, A61L 31/08, A61L 31/10, A61L 31/14, A61P 9/06

(54) **FORMATION OF THERAPEUTIC SCAR USING SMALL PARTICLES**
BILDUNG EINER THERAPEUTISCHEN NARBE MIT KLEINEN TEILCHEN
FORMATION D'UNE CICATRICE THÉRAPEUTIQUE AVEC DES PETITES PARTICULES

(30) Priority: 09.05.2006 US 799122 P
(43) Date of publication of application: 21.01.2009
(73) Proprietor: Syntach AG, 8200 Schaffhausen (CH)
(72) Inventor: CORNELIUS, Richard, Wayzata, Minnesota 55391 (US); QUINT, Bodo, 72108 Rottenburg (DE); SEIBOLD, Gerd, 72119 Ammerbuch (DE); JOERGENSEN, Ib Erling, 72401 Haigerloch (DE); NIELSEN, Stevan, 72108 Rottenburg Am Neckar (DE)
(74) Representative: KIPA AB
(86) International application number: PCT/EP2007/054450
(87) International publication number: WO 2007/128818

(56) References cited:
- WO-A-01/64123
- WO-A-03/003948
- WO-A-2006/042246
- WO-A-2007/055397
- US-A- 4 638 803
- WILLERT H G: "Reactions of the articular capsule to wear products of artificial joint prostheses." JOURNAL OF BIOMEDICAL MATERIALS RESEARCH, vol. 11, no. 2, March 1977 (1977-03), pages 157-164, XP002452733 ISSN: 0021-9304

## Description

### Background of the Invention

This application generally relates to techniques for scarring a desired tissue location in the human body. These techniques can be implemented through devices such as those described in U.S. Patent No. 7,097,643 filed March 2, 2004; and U.S. Publication Nos. 2004-0220655 filed March 2, 2004; 2006-0116666 filed October 7, 2005; and 2004-0254597 filed April 30, 2004, in addition to the specified applications below, and in other possible delivery modes as described in this specification.

In application USA4638803 a catheter based inflatable balloon is described on to which a microparticle coating comprising of metals such as iron or copper is placed. The microparticles in this disclosure are conceived to induce the formation of a thrombosis, with subsequent invasion by capillaries and fibroblasts and scar tissue formation.

An earlier work published in the Journal of Biomedical Materials Research 1977, 11, 157-164, has previously shown that metal and plastic granules can give rise to undesired scar tissue formation through phagocytosis and granulation, primarily though in the joint tissue.

The application of WO2006/042246A, teaches a method and apparatus of causing deep scar formation, using degradable spheres which are coated and/or encapsulate a drug, alkylating agent, an antibiotic, biodegradable polymer, or other material such as metallic copper or copper containing compounds being embedded in a biodegradeable matrix.

The previously noted applications describe devices that create clinically beneficial scarring at target locations in the human body. For example, this scar generation may be used for treatment of heart arrhythmias such as atrial fibrillation or treatment of certain cancers. In a more specific example, one such device creates scar tissue around the pulmonary vein ostium for treatment of atrial fibrillation. This ring of scar tissue stops transmission of electrical potentials from the pulmonary vein to the atrial tissue, thereby reducing or stopping arrhythmia.

Furthermore it has also been described in WO2001/64123A, that transmural lesions in the pulmonary vein can be performed using a balloon catheter which utilizes titanium or aluminium oxide particles to scatter visible or near-infrared radiation. However in this application the particles were bound permanently to the implant and should therefore not be released into the targeted tissue.

The prior art describes different mechanisms for producing therapeutic scarring, such as mechanical pressure, energy ablation, inflammatory materials, ablative drugs and combinations thereof.

In some clinical applications, such as for electrical isolation of the pulmonary veins, it is important for the scar to extend through the full thickness of the tissue wall ("transmural" scarring) to yield the desired electrical isolation. However, prior art mechanisms may not achieve this transmural or deep tissue scarring necessary for some procedures (e.g., arrhythmia treatments). Hence, an improved method or apparatus for achieving such scarring would be advantageous.

### SUMMARY

Accordingly, embodiments of the present invention preferably seek to mitigate, alleviate or eliminate one or more deficiencies, disadvantages or issues in the art, such as the above-identified, singly or in any combination by providing a method and apparatus according to the appended patent claims.

One aspect of the present invention describes the use of small particles, such as micro particles or nanoparticles, to produce a therapeutic scar such as "trans-mural" scarring or other desired "deep tissue" scarring. In an embodiment, these particles may be delivered to a target location by an implant. More specifically, these particles may be incorporated into the structure of implants or into the coatings on implants. In other preferred embodiments, these particles may directly be delivered by electrophoresis or hydraulic injection into the target tissue.

In an aspect of the invention, a method of generating a substantially transmural scar in mammalian tissue is provided. The method comprises providing a plurality of particles, the particles sized for causing an inflammatory response in the mammalian tissue; introducing the particles into the mammalian tissue; causing the particles to disburse substantially into a thickness of the mammalian tissue; allowing the particles to inflame the tissue until a substantially transmural scar is generated in the mammalian tissue.

In a further aspect of the invention, an apparatus for causing a substantially transmural scar in body tissue is provided. The apparatus comprises a plurality of particles; a delivery device for introducing said particles to said body tissue; a mechanism for disbursing said particles substantially through a thickness of said body tissue; wherein said particles are sufficiently sized to cause a substantially transmural scar in said body tissue.

Further embodiments of the invention are defined in the dependent claims, wherein features for the different aspects of the invention apply mutatis mutandis.

It should be emphasized that the term "comprises/comprising" when used in this specification is taken to specify the presence of stated features, integers, steps or components but does not preclude the presence or addition of one or more other features, integers, steps, components or groups thereof.

### brief description of the drawings

These and other aspects, features and advantages of which embodiments of the invention are capable of will be apparent and elucidated from the following description of embodiments of the present invention, reference being made to the accompanying drawings, in which
Figure 1 illustrates a side view of a preferred embodiment of an implant according to a preferred embodiment of the present invention;
Figure 2A illustrates a end view of a preferred embodiment of an implant within an ostium of a pulmonary vein according to the present invention; and
Figure 2B illustrates an enlarged view of the implant of Figure 2A.

### DETAILED DESCRIPTION OF THE INVENTION

Specific embodiments of the invention now will be described with reference to the accompanying drawings. This invention may, however, be embodied in many different forms and should not be construed as limited to the embodiments set forth herein; rather, these embodiments are provided so that this disclosure will be thorough and complete. The terminology used in the detailed description of the embodiments illustrated in the accompanying drawings is not intended to be limiting of the invention. One aspect of this invention relates to how the body handles different sizes of foreign materials. In general, the body can handle perceived foreign materials in two ways. If the foreign material is relatively large, the body will respond by attempting to isolate the foreign material from the body by encasing it in scar tissue. This occurs with an inflammatory response around the surface of the foreign material which heals into an encasing layer of scar. If the foreign material is smaller, for example on the scale of a cell, the body will respond by trying to absorb and digest or "eat" the foreign material, as it does with virus.

This latter response may be used for treatment purposes by introducing materials which are at or below the size of cells, such as microparticles or nanoparticles. When delivered to a desired target location, for example by way of implants or coatings on implants, these particles are believed to promote scarring through macrophage/phagocytosis activation, often causing cell death of macrophages that attempt "digestion". The death of these macrophage cells may result in inflammation deeper in the wall of the tissue from where the foreign material was originally deposited. Further, the inflammatory response is not limited to the identified foreign body, but also to the surrounding tissue. Phagocytosis and fibrosis (scarring) are concurrent inflammatory response which cause a deeper tissue scarring effect. Additionally, prior to death, the macrophage cell may transport the foreign material deeper into the tissue and thereby further increase the depth of the inflammatory response. The end result of this reaction is deeper and more aggressive scar generation which may be synergistically used with other scarring techniques (e.g., tissue cutting).

It should be also noted that in addition to size, other factors may influence the biological response, such as morphology, surface charge and area. With regard to particle morphology, nanofibers such as asbestos or carbon nanotubes are known to cause intensive and potential uncontrolled inflammatory response. They offer a very high surface area which is believed to be the cause for the identification as a foreign substance. Further, due to agglomeration, these materials can be difficult to be cleared by phagocytosis. Clay-like materials can offer the beneficial aspect ratio of fiber-like substances but due to their natural surface charge they separate in aqueous solutions and can be cleared by phagocytosis. This kind of additive is beneficial in order to create a controlled inflammatory response. In general, particle sizes greater than 1 µm can be difficult to be cleared by phagocytosis and in this example, signal substances are transmitted from phagocytes in order to stimulate more intensive fibrosis (scar generation) and phagocytosis.

With regard to surface properties, the amount of adsorption that is often key for the foreign body identification is dependent, at least in part, on surface area, surface charge and dispersive surface effects. In general, positive charged surfaces are less biocompatible due to effective adsorption of negatively charged body fluid contents. Most metal oxides and ceramic surfaces offer a more biocompatible surface due to their natural negative surface charge. Materials with a very low surface polarity and charge, such as PE and PTFE which are known to be highly biocompatible.

A further example factor that may influence the biological response is radiation. It is well known that radiation can directly cause cell death/necrosis. Therefore the use of radioactive particles may lead to long term identification as a foreign body.

Yet another example factor that may influence the biological response of the body is the use of enzymes, either as "foreign" particles or in addition to particles. During an inflammatory reaction, the body requires increased amounts of fibrin, plasmin, thrombin and kinins. However, the primary response to inflammation is through the immune system and its increased use of enzymes in order to support these raw materials. Localized deficiencies of enzymes can prolong inflammation and delay healing. Oral use of proteolytic enzymes may reduce healing times by up to 50 percent. In addition to proteases, amylases and lipases also play a role in the inflammatory response. Each contributes in specific functions to assist the inflammatory responses. These enzymes can be gathered from several sources and concentrated for supplemental use. The use of fungal enzymes typically function in a broader pH range than those taken from beef and pork (so-called pancreatin). An important consideration may be that there are specific pH changes in tissue during inflammation so that the affected tissue comes either more alkaline or more acidic than normal. Plant enzymes have a broader range of substrates that can be used. Further, digestive enzymes from the pancrease (e.g., collagenases and proteases) are selective in the bonds they can hydrolyze and therefore digest.

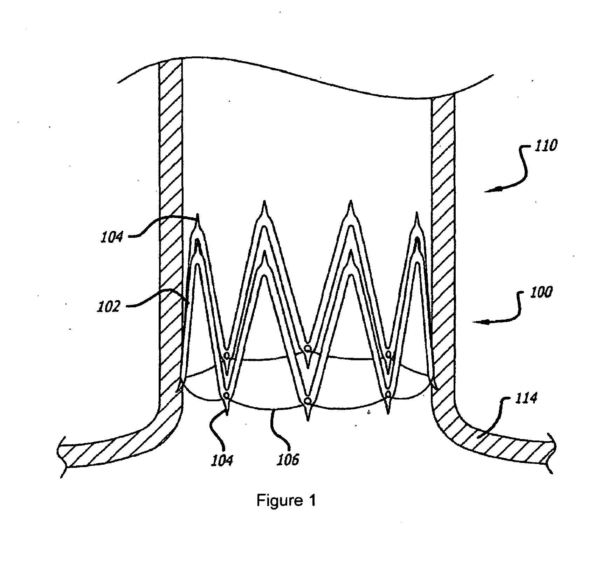

Figure 1 illustrates an embodiment of a self expanding mechanical implant 100 according to the present invention located near an ostium 114 of a pulmonary vein 110. The implant includes a plurality of struts 102 that are configured to exert a mechanical pressure against the desired target tissue. The peaks where each strut 102 connects to the next strut 102 includes an anchoring barb 104 which is shaped to pierce the target tissue and therefore provide anchoring support to the implant 100. The implant 100 optionally includes a wire 106 that is fixed to the peak of the struts 102 on one end of the implant 100 for creating a narrow band of pressure on the target tissue. Further details of the self expanding mechanical implant 100, and similar Nitinol implants, can be seen in U.S. Publication No. 2004-0220655 filed March 2, 2004 and U.S. Publication No. 2006-0116666 filed October 7, 2005.

The implant 100 is further coated with a biodegradable polymer such as 50/50 PLGA loaded with 50% by weight of 2 micron copper particles. Preferably, the 50/50 PLGA will have largely or completely degraded after 30 days thereby freeing the 2 micron particles to direct exposure to the tissue. After 30 days there will be significantly more inflammation around the implant than would be possible with no coating, only a PLGA coating or even only a solid copper plating. Further, this inflammation caused by the implant 100 will extend much deeper into the tissue wall than for copper plated devices.

With additional time, inflammation from the implant 100 resolves into a scar yielding the desired deeper scarring from the deeper inflammation generated by the small particle copper loaded coating. It is believed that the body's digestive immune response (phagocytosis) to the foreign material occurs with particles that are as large as 10-20 microns and be fully evident as the particle size reduces to 5 micron or less. Further, an inflammation response is also possible with the use of even smaller particles such as nanoparticles. Additional information about nanoparticles can be found in Nanotechnology: A Brief Literature Review by M. Ellin Doyle, Ph.D., June 2006 University of Wisconsin FRI Briefings and in Industrial Application of Nanomaterials - Chances and Risks, Wolfgang Luther (ed.), VDI Technologiezentrum GmbH.

This previously described principal (i.e., a bodily immune response to small particles) may according to certain embodiments be applied to other materials as well (e.g., materials with other mechanisms of causing inflammation). Moreover, the total amount of the inflammatory particles introduced may be varied through control of the coating thickness or the percent weight loading of the particles in coating. In this respect, the thickness of scar generation may be adjusted to achieve a desired result.

Further, the inflammatory particles may be incorporated to be released in predetermined quantities over a period of time. For example, inflammatory materials may be filled, coumpounded or otherwise incorporated into additional materials that degrade within the body after predetermined times, thereby delaying inflammatory reactions after implantation. In another embodiment, such time release techniques may be used to release constant amounts of inflammatory materials over an extended period of time or provide multiple "spikes" of inflammatory material at predetermined times after delivery of a device within a patient.

In another embodiment, the scar generating material may be a drug such as Actinomycin, FUDR or Vinblastin as examples. These drugs can be encapsulated in biodegradable micro-spheres having diameters of less than about 5 microns made of materials such as, but not limited to, PLGA, PLA, Polyanhydride, or chitosan. The micro-sphere material may also be made of a durable material which allows diffusion of the encapsulated drug. These micro-spheres may then be coated directly to the surface of an implant or adhered to the implant with a binder material.

The construction of this embodiment allows the micro-spheres to diffuse or to be carried by macrophages deeper into the tissue before delivering the encapsulated drug or material. Thus, this technique may achieve deeper scarring as compared to direct delivery. Further, the encapsulated drug itself (i.e., not just the size of the drug particle) may create a more intense inflammatory response and therefore create more dense scar tissue.

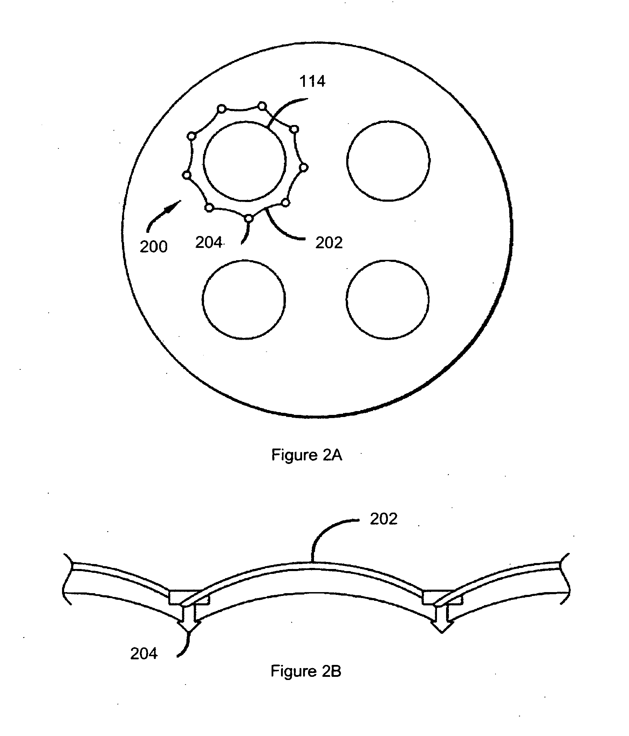

Figures 2A and 2B illustrate another embodiment of an implant 200 that delivers inflammatory particles via a strand 202 made with a biodegradable polymer matrix. The strand 202 is connected to a plurality of staples 204. The staples 204 are punctured into the tissue, fastening the strand 202 against the target tissue. Further details of this implant and example delivery systems can be seen in U.S. Patent No. 7,097,643 filed March 2, 2004, (e.g. figures 21 a and 21 b) and U.S. Publication No. 2004-0220655, filed March 2, 2004 fig. 21. The delivery device feeds out lengths of the particle loaded strand 202 with staples 204 attached at intervals along the length (e.g., every 5 millimeters).

In this way, the steerable delivery catheter may be maneuvered over the desired path of scar generation, leaving behind a line of the particle containing strand 202. This path may be visualized during the procedure using either radioopaque markers incorporated into the staples 204 or radioopaque loading of the strand material.

In another embodiment, the staples of the previously described embodiment are formed of a superelastic and/or shape memory material, such as a polymer or metal, for instance Nitinol, in an open ring shape with the ends being in close proximity to each other (e.g., a "C" shape). As the staple feeds out the tip of a delivery catheter, the ends of the staples are flexed apart and are pressed against the tissue by the tip of the catheter. In this way, when the staple is released, the ends spring back together and embed in the tissue at the targeted site. This anchors the strand to the tissue at this point. Alternatively, another mechanism of adhering the strand to the tissue surface is a bio-adhesive.

In additional preferred embodiments, inflammatory particles (e.g., microparticles or nanoparticles) may be released from materials such as filled degradable polymers, crystallites of degradable semi crystalline plastics, contents of plastic blends, copolymers, fiber containing materials, and carbon fibers. Further, instead of a matrix based on degradable polymers, degradable ceramics or degradable metals may be used.

In another embodiment, the particles may be delivered directly into the tissue at the targeted site without any additional implant using direct injection of the particles. This may be performed using the technique of electrophoresis on a charged particles surface or particle coating to enable the electrophoreretic delivery. An electrode at the end of the catheter drives the particles away from the catheter tip and into the tissue when the electrode is polarized. This basic technology is well known and examples are shown in patents 4,411,648; 5,807,306; 5,704,908; and 6,219,577.

The direct delivery of the particles (e.g., microparticles or nanoparticles) into the tissue may also be accomplished using hydraulic pressure for particle injection. This technique is also a commonly known method, whereby the particles are injected using fluid pressure either through micro-needle(s) or nozzle(s) at the tip of the catheter or around the circumference of a centering device, for example placed in the ostium of the pulmonary vein. Examples of devices to enable this type of delivery are shown in patents 5,306,250; 5,538,504; or 6,254,573.

In another embodiment, carbon fibers may be used to cause inflammation and enhance the modulus of composites (e.g., by enhancing the forces provided by a mechanical implant). The carbon fibers may be incorporated into a resorbable matrix, thereby offering a high surface area that can be identified as a foreign body. The inflammatory reaction of carbon fibers has been described in some literature as "low and not critical". However, using, for example, textile fiber finishing methods or plasma treatment, the surface charge and polarity may be modified. Thus, due to their mechanical reinforcement and low inflammatory reaction, carbon fibers may synergistically compliment the effects of traditional scar generating techniques (e.g., cutting or pressure).

## Claims

1. An apparatus adapted to cause a desired transmural scar in a body tissue, said apparatus comprising:
a plurality of particles ;
a delivery device configured to introduce said particles into said body tissue;
a mechanism for disbursing said particles through a thickness of said body tissue;
said particles being adapted to cause said transmural scar in said body tissue, wherein said particles are less than 20 microns in size.

2. The apparatus of claim 1, wherein said particles are inflammatory particles, and wherein a total amount of said inflammatory particles is controlled by a thickness of a coating of said delivery device or percent weight loading of the inflammatory particles in said coating, such that a thickness of scar generation is target adjustable.

3. The apparatus of claim 1, wherein said particles are inflammatory particles that are incorporated in said delivery device to be released in predetermined quantities over a period of time; or wherein said particles are inflammatory particles that are incorporated in said delivery device to be released in predetermined quantities over a period of time, and wherein said inflammatory particles are incorporated into additional materials that degrade within the body after predetermined times, such that inflammatory reactions are delayed after delivery; or wherein said particles are inflammatory particles that are incorporated in said delivery device to be released in predetermined quantities over a period of time, and wherein said apparatus comprising time release mechanisms in use of said device releasing constant amounts of inflammatory particles over an extended period of time or providing multiple spikes of inflammatory particles at predetermined times after delivery of said delivery device within a patient.

4. The apparatus of claim 1, wherein said particles comprise a drug encapsulated in biodegradable micro-spheres having diameters of less than about 5 microns, and wherein said particles are made of PLGA, PLA, Polyanhydride, or chitosan; or wherein said particles comprise a drug encapsulated in biodegradable micro-spheres having diameters of less than about 5 microns, and wherein said particles are made of PLGA, PLA, Polyanhydride, or chitosan, and wherein said micro-sphere material are made of a durable material which allows diffusion of the encapsulated drug; or wherein said particles comprise a drug encapsulated in biodegradable micro-spheres having diameters of less than about 5 microns, and wherein said particles are made of PLGA, PLA, Polyanhydride, or chitosan, and wherein said micro-spheres are coated to the surface of an implant or adhered to said implant with a binder material.

5. The apparatus of claim 1, wherein said delivery device is an implant (200) arranged to said particles via a strand (202) made with a biodegradable polymer matrix, wherein said strand (202) is connected to a plurality of staples (204) arranged to be punctured into said body tissue, in use of the apparatus fastening the strand (202) against said body tissue; or wherein said delivery device is an implant (200) arranged to said particles via a strand (202) made with a biodegradable polymer matrix, wherein said strand (202) is connected to a plurality of staples (204) arranged to be punctured into said body tissue, in use of the apparatus fastening the strand (202) against said body tissue and wherein said staples (204) comprise radioopaque markers, or said strand (202) comprises a material having a radioopaque loading, and/or wherein said staples are formed of a superelastic material.

6. The apparatus of claim 1, wherein said particles are inflammatory particles, that are arranged in filled degradable polymers, crystallites of degradable semi crystalline plastics, contents of plastic blends, copolymers, fiber containing materials, and carbon fibers; or wherein said particles are inflammatory particles, arranged in degradable ceramics or degradable metals.

7. The apparatus of claim 1, wherein said delivery device comprises an implant, or a self expanding mechanical implant; or wherein said delivery device comprises an implant, or a self expanding mechanical implant, and wherein said implant further comprises a coating, said coating containing said particles; or wherein said delivery device comprises an implant, or a self expanding mechanical implant, and wherein said implant further comprises a coating, said coating containing said particles, and wherein said coating is arranged to degrade within a body over a predetermined time.

8. The apparatus of any of claim 7, wherein said implant contains said particles.

9. The apparatus of any of claims 1 or 7, wherein said particles are copper.

10. The apparatus of claim 1, wherein said particles are fiber, or carbon fiber; or wherein said particles are encapsulated in micro-spheres; or wherein said delivery device is an electrophoresis delivery device; or wherein said delivery device is a hydraulic delivery device; or wherein said particles are nanoparticles.

11. The apparatus of claim 1, wherein said particles are less than 5 microns in size.

12. Particles less than 20 microns in size for use in the treatment of heart arrhythmias, wherein said use comprises generating a transmural scar in mammalian tissue with said particles causing an inflammatory response in said mammalian tissue when said particles are introduced into said mammalian tissue and disbursed into a thickness of said mammalian tissue to inflame said tissue until said transmural scar is generated in said mammalian tissue.

13. Particles for the use of claim 12, wherein said particles are less than 5 microns in size, or wherein said particles are nanoparticles.

14. Particles for the use of any of claims 12 to 13, wherein said introduced particles are delivered with an implant implanted at a target location within a mammalian body, and wherein said particles disbursed into a thickness of said mammalian tissue are released particles from said implant or from a coating on said implant.

15. Particles for the use of any of the preceding claims 12 to 14, wherein said particles introduced into mammalian tissue further comprises that said particles are delivered in micro-spheres; or wherein said said particles are introduced into mammalian tissue further by a force applied on a charged coating on said particles with electrophoresis; or wherein said particles introduced into mammalian tissue further by a hydraulic pressure applied on said particles.

## Patentansprüche

1. Gerät, das zur Bildung einer gewünschten transmuralen Narbe in einem Körpergewebe angepasst ist, wobei das Gerät umfasst:
eine Vielzahl von Teilchen;
ein Zuführgerät, das für die Einführung der Teilchen in das Körpergewebe konfiguriert ist;
einen Mechanismus zur Ausgabe der Teilchen durch eine Dicke des Körpergewebes,
wobei diese Teilchen so angepasst sind, dass sie transmurale Narbe in dem Körpergewebe bilden, wobei die Teilchen ein Größe von weniger als 20 Mikron aufweisen.

2. Gerät gemäß Anspruch 1, wobei es sich bei den Teilchen um inflammatorische Teilchen handelt, und wobei eine Gesamtmenge der inflammatorischen Teilchen durch die Dicke einer Beschichtung des Zuführgerätes oder die prozentuale Gewichtsbelastung der inflammatorischen Teilchen in der Beschichtung reguliert wird, so dass eine Zieldicke der Narbenbildung eingestellt werden kann.

3. Gerät gemäß Anspruch 1, wobei es sich bei den Teilchen um inflammatorische Teilchen handelt, die in dem Zuführgerät integriert sind, so dass sie in zuvor festgelegt Mengen über einen Zeitraum hinweg freigesetzt werden können; oder wobei es sich bei den Teilchen um inflammatorische Teilchen handelt, die in dem Zuführgerät integriert sind, so dass sie über einen Zeitraum in zuvor festgelegten Mengen freigesetzt werden können, und wobei die inflammatorischen Teilchen in zusätzlichen Materialen integriert sind, welche im Laufe einer zuvor festgelegten Zeit im Körper abgebaut werden, so dass inflammatorische Reaktionen infolge der Zuführung verzögert werden; oder wobei es sich bei den Teilchen um inflammatorische Teilchen handelt, die in dem Zuführgerät integriert sind, um in zuvor festgelegten Mengen über einen Zeitraum hinweg freigesetzt zu werden, und wobei das Gerät über Time-Release-Mechanismen verfügt, die bei Verwendung des Geräts gleichbleibende Mengen von inflammatorischen Teilchen über einen längeren Zeitraum freisetzen oder nach der Zuführung des Zuführgeräts im Körper eines Patienten an zuvor festgelegten Zeitpunkten mehrere Spitzen von inflammatorischen Teilchen liefern.

4. Gerät gemäß Anspruch 1, wobei die Teilchen ein Medikament enthalten, das in biologisch abbaubaren Mikrosphären eingekapselt ist, welche Durchmesser von weniger als ungefähr 5 Mikron aufweisen, und wobei die Teilchen aus PLGA, PLA, Polyanhydrid oder Chitosan bestehen; oder wobei die Teilchen ein Medikament umfassen, das in biologisch abbaubaren Mikrosphären eingekapselt ist, welche Durchmesser von weniger als ungefähr 5 Mikron aufweisen, und wobei die Teilchen aus PLGA, PLA, Polyanhydrid oder Chitosan bestehen, und wobei es sich bei dem Mikrosphärenmaterial um ein strapazierbares Material handelt, das die Diffusion des eingekapselten Medikaments ermöglicht; oder wobei die Teilchen ein Medikament umfassen, das in biologisch abbaubaren Mikrosphären eingekapselt ist, welche Durchmesser von weniger als ungefähr 5 Mikron aufweisen, und wobei die Teilchen aus PLGA, PLA, Polyanhydrid oder Chitosan bestehen, und wobei die Mikrosphären auf der Oberfläche eines Implantats aufgebracht oder unter Verwendung eines Bindemittels an das Implantat angehaftet sind.

5. Gerät gemäß Anspruch 1, wobei es sich bei dem Zuführgerät um ein Implantat (200) handelt, das über einen Strang (202), welcher aus einer biologisch abbaubaren Polymermatrix besteht, an die Teilchen angeordnet ist, wobei der Strang (202) mit einer Vielzahl von Klammern (204) verbunden ist, die so angeordnet sind, dass sie bei der Benutzung des Geräts in das Körpergewebe eingestochen werden können, so dass der Strang (202) am Körpergewebe befestigt wird; oder wobei es sich bei dem Zuführgerät um ein Implantat (200) handelt, das über einen Strang (202), welcher aus einer biologisch abbaubaren Polymermatrix besteht, an die Teilchen angeordnet ist, wobei der Strang (202) mit einer Vielzahl von Klammern (204) verbunden ist, die so angeordnet sind, dass sie bei der Benutzung des Geräts in das Körpergewebe eingestochen werden können, so dass der Strang (202) am Körpergewebe befestigt wird und wobei die Klammern (204) strahlenundurchlässige Marker umfassen, oder der Strang (202) ein Material umfasst, das eine radio-opake Ladung hat und/oder die Klammern aus einem superelastischen Material bestehen.

6. Gerät gemäß Anspruch 1, wobei es sich bei den Teilchen um inflammatorische Teilchen handelt, die in gefüllten abbaubaren Polymeren, Kristalliten von abbaubaren teilkristallinen Kunststoffen, Inhaltsstoffen von Kunststoffgemischen, Copolymeren, faserigen Materialien und Carbonfasern angeordnet sind; oder wobei es sich bei den Teilchen um inflammatorische Teilchen handelt, die in abbaubaren keramischen Materialien oder abbaubaren Metallen angeordnet sind.

7. Gerät gemäß Anspruch 1, wobei das Zuführgerät ein Implantat oder ein selbstexpandierbares mechanisches Implantat umfasst; oder wobei das Zuführgerät ein Implantat, oder ein selbstexpandierbares mechanisches Implantat umfasst, und wobei das Implantat ferner eine Beschichtung umfasst, wobei die Teilchen in der Beschichtung enthalten sind; oder wobei das Zuführgerät ein Implantat oder ein selbstexpandierbares mechanisches Implantat umfasst, und wobei das Implantat ferner eine Beschichtung aufweist, wobei die Teilchen in der Beschichtung enthalten sind, und wobei die Beschichtung so angeordnet ist, dass sie im Laufe einer zuvor festgelegten Zeitspanne im Körper abgebaut wird.

8. Gerät gemäß Anspruch 7, wobei die Teilchen im Implantat enthalten sind.

9. Gerät gemäß einem beliebigen der Ansprüche 1 oder 7, wobei es sich bei den Teilchen um Kupfer handelt.

10. Gerät gemäß Anspruch 1, wobei es sich bei den Teilchen um Fasern oder Carbonfasern handelt; oder wobei die Teilchen in Mikrosphären eingekapselt sind; oder wobei es sich bei dem Zuführgerät um ein Elektrophorese-Zuführgerät handelt; oder wobei es sich bei dem Zuführgerät um ein hydraulisches Zuführgerät handelt; oder wobei es sich bei den Teilchen um Nanoteilchen handelt.

11. Gerät gemäß Anspruch 1, wobei die Teilchen ein Größe von weniger als 5 Mikron haben.

12. Teilchen von weniger als 20 Mikron Größe zum Einsatz in der Behandlung von Herzrhythmusstörungen, wobei der Einsatz die Erzeugung einer transmuralen Narbe in Säugetiergewebe umfasst, wobei die Teilchen eine inflammatorische Reaktion in dem Säugetiergewebe hervorrufen, wenn die Teilchen in das Säugetiergewebe eingeführt und in einer Dicke des Säugetiergewebes ausgegeben werden, um in dem Gewebe eine Entzündung hervorzurufen, bis die transmurale Narbe in dem Säugetiergewebe erzeugt wird.

13. Teilchen zum Einsatz gemäß Anspruch 12, wobei die Teilchen eine Größe von weniger als 5 Mikron aufweisen, oder wobei es sich bei den Teilchen um Nanoteilchen handelt.

14. Teilchen zum Einsatz gemäß Ansprüchen 12 bis 13, wobei die eingeführten Teilchen mittels eines Implantats zugeführt werden, das an einer Zielstelle innerhalb eines Säugetierkörpers implantiert wird, und wobei es sich bei den Teilchen, die in einer Dicke des Säugetiergewebes ausgegeben werden, um von dem Implantat oder von der Beschichtung des Implantats freigesetzte Teilchen handelt.

15. Teilchen zum Einsatz gemäß einem beliebigen der vorangegangenen Ansprüche 12 bis 14, wobei das Einbringen der Teilchen in das Säugetiergewebe ferner umfasst, dass die Teilchen in Mikrosphären zugeführt werden; oder wobei die Teilchen ferner durch eine Krafteinwirkung in das Säugetiergewebe eingebracht werden, die auf einer geladenen Beschichtung mittels Elektrophorese auf die Teilchen ausgeübt wird; oder wobei die Teilchen ferner durch einen auf die Teilchen ausgeübten hydraulischen Druck in das Säugetiergewebe eingeführt werden.

## Revendications

1. Appareil adapté afin de provoquer une cicatrice transmurale désirée dans un tissu corporel, ledit appareil comprenant :
une pluralité de particules ;
un dispositif de délivrance configuré afin d'introduire lesdites particules dans ledit tissu corporel ;
un mécanisme de dispersement desdites particules à travers une épaisseur dudit tissu corporel ;
lesdites particules étant adaptées afin de provoquer ladite cicatrice transmurale dans ledit tissu corporel, où lesdites particules présentent une taille inférieure à 20 microns.

2. Appareil selon la revendication 1, dans lequel lesdites particules sont des particules inflammatoires, et dans lequel une quantité totale desdites particules inflammatoires est contrôlée par une épaisseur d'un revêtement dudit dispositif de délivrance ou une charge en pourcent en poids des particules inflammatoires dans ledit revêtement, de sorte qu'une épaisseur de génération de cicatrice est ajustable à une cible.

3. Appareil selon la revendication 1, dans lequel lesdites particules sont des particules inflammatoires qui sont incorporées dans ledit dispositif de délivrance afin d'être libérées en quantités prédéterminées sur une période de temps ; ou dans lequel lesdites particules sont des particules inflammatoires qui sont incorporées dans ledit dispositif de délivrance afin d'être libérées en quantités prédéterminées sur une période de temps, et dans lequel lesdites particules inflammatoires sont incorporées dans des matières supplémentaires qui se dégradent à l'intérieur du corps après des durées prédéterminées, de telle sorte que des réactions inflammatoires sont différées après délivrance ; ou dans lequel lesdites particules sont des particules inflammatoires qui sont incorporées dans ledit dispositif de délivrance afin d'être libérées en quantités prédéterminées sur une période de temps, et où ledit appareil comprend des mécanismes de libération contrôlée lors de l'utilisation dudit dispositif libérant des quantités constantes de particules inflammatoires sur une période de temps prolongée ou fournissant plusieurs pics de particules inflammatoires à des moments prédéterminés après délivrance dudit dispositif de délivrance à l'intérieur d'un patient.

4. Appareil selon la revendication 1, dans lequel lesdites particules comprennent un médicament encapsulé dans des microsphères biodégradables présentant des diamètres inférieurs à environ 5 microns, et dans lequel lesdites particules sont composées de PLGA, de PLA, de polyanhydride ou de chitosane ; ou dans lequel lesdites particules comprennent un médicament encapsulé dans des microsphères biodégradables présentant des diamètres inférieurs à environ 5 microns, et dans lequel lesdites particules sont composées de PLGA, de PLA, de polyanhydride ou de chitosane, et dans lequel lesdites matières des microsphères sont composées d'une matière durable qui permet une diffusion du médicament encapsulé ; ou dans lequel lesdites particules comprennent un médicament encapsulé dans des microsphères biodégradables présentant des diamètres inférieurs à environ 5 microns, et dans lequel lesdites particules sont composées de PLGA, de PLA, de polyanhydride ou de chitosane, et dans lequel lesdites microsphères revêtent la surface d'un implant ou adhèrent audit implant grâce à une matière liante.

5. Appareil selon la revendication 1, dans lequel ledit dispositif de délivrance est un implant (200) disposé par rapport auxdites particules via un brin (202) composé d'une matrice polymère biodégradable, où ledit brin (202) est relié à une pluralité d'agrafes (204) disposées de manière à perforer ledit tissu corporel, lors de l'utilisation de l'appareil fixant le brin (202) contre ledit tissu corporel ; ou dans lequel ledit dispositif de délivrance est un implant (200) disposé par rapport auxdites particules via un brin (202) composé d'une matrice polymère biodégradable, où ledit brin (202) est relié à une pluralité d'agrafes (204) disposées de manière à perforer ledit tissu corporel, lors de l'utilisation de l'appareil fixant le brin (202) contre ledit tissu corporel et dans lequel lesdites agrafes (204) comprennent des marqueurs radio-opaques, ou ledit brin (202) comprend une matière présentant une charge radio-opaque, et/ou dans lequel lesdites agrafes sont composées d'une matière super-élastique.

6. Appareil selon la revendication 1, dans lequel lesdites particules sont des particules inflammatoires, qui sont disposées dans des polymères dégradables chargés, des cristallites de matières plastiques semi-cristallines dégradables, des contenus de mélanges de matières plastiques, des copolymères, des matières fibreuses, et des fibres de carbone ; ou dans lequel lesdites particules sont des particules inflammatoires, disposées dans des céramiques dégradables ou des métaux dégradables.

7. Appareil selon la revendication 1, dans lequel ledit dispositif de délivrance comprend un implant, ou un implant mécanique auto-déployable ; ou dans lequel ledit dispositif de délivrance comprend un implant, ou un implant mécanique auto-déployable, et dans lequel ledit implant comprend en outre un revêtement, ledit revêtement contenant lesdites particules ; ou dans lequel ledit dispositif de délivrance comprend un implant, ou un implant mécanique auto-déployable, et dans lequel ledit implant comprend en outre un revêtement, ledit revêtement contenant lesdites particules, et dans lequel ledit revêtement est disposé afin de se dégrader à l'intérieur d'un corps sur une durée prédéterminée.

8. Appareil selon la revendication 7, dans lequel ledit implant contient lesdites particules.

9. Appareil selon l'une quelconque des revendications 1 à 7, dans lequel lesdites particules sont du cuivre.

10. Appareil selon la revendication 1, dans lequel lesdites particules sont une fibre, ou une fibre de carbone ; ou dans lequel lesdites particules sont encapsulées dans des microsphères ; ou dans lequel ledit dispositif de délivrance est un dispositif de délivrance par électrophorèse ; ou dans lequel ledit dispositif de délivrance est un dispositif de délivrance hydraulique ; ou dans lequel lesdites particules sont des nanoparticules.

11. Appareil selon la revendication 1, dans lequel lesdites particules présentent une taille inférieure à 5 microns.

12. Particules d'une taille inférieure à 20 microns destinées à être une utilisation dans le traitement d'arythmies cardiaques, où ladite utilisation comprend une génération d'une cicatrice transmurale dans un tissu mammalien avec lesdites particules causant une réponse inflammatoire dans ledit tissu mammalien lorsque lesdites particules sont introduites dans ledit tissu mammalien et dispersées dans une épaisseur dudit tissu mammalien afin de provoquer une inflammation dudit tissu jusqu'à ce que ladite cicatrice transmurale soit générée dans ledit tissu mammalien.

13. Particules destinées à l'utilisation prévue par la revendication 12, où lesdites particules présentent une taille inférieure à 5 microns, ou où lesdites particules sont des nanoparticules.

14. Particules destinées à l'utilisation prévue par l'une quelconque des revendications 12 à 13, où lesdites particules introduites sont délivrées avec un implant implanté à un emplacement cible à l'intérieur d'un corps mammalien, et où lesdites particules dispersées dans une épaisseur dudit tissu mammalien sont des particules libérées dudit implant ou d'un revêtement sur ledit implant.

15. Particules destinées à l'utilisation prévue par l'une quelconque des revendications précédentes 12 à 14, où lesdites particules introduites dans un tissu mammalien comprennent en outre lesdites particules qui sont délivrées dans des microsphères ; ou où lesdites particules sont en outre introduites dans un tissu mammalien par une force appliquée sur un revêtement chargé sur lesdites particules par électrophorèse ; ou où lesdites particules sont en outre introduites dans un tissu mammalien par une pression hydraulique appliquée sur lesdites particules.
